# EUROPEAN PATENT APPLICATION

(11) **EP 3 903 706 A1**
(43) Date of publication of application: **03.11.2021**
(21) Application number: 19903910.8
(22) Date of filing: 25.12.2019
(51) Int. Cl.: A61B 18/02

(54) **CRYOABLATION CATHETER**

(30) Priority: 25.12.2018 CN 201811593755
(71) Applicant: Synaptic Medical (Beijing) Co. Ltd., Beijing 100111 (CN)
(72) Inventor: PENG, Bo, Beijing 101111 (CN); GONG, Jie, Beijing 101111 (CN); FENG, Ji, Beijing 101111 (CN)
(74) Representative: Handsome I.P. Ltd
(86) International application number: PCT/CN2019/128137
(87) International publication number: WO 2020/135468

(57) **Abstract**

The present invention discloses a cryoablation catheter, including: a main tube having a distal end, a proximal end, and at least one lumen; an expansion element, with which the distal end of the main tube is sleeved; a fluid delivery tube extending into an expansion element through the lumen of the main tube, an opening being provided at a distal end of the fluid delivery tube, and a cryogenic fluid being ejected out through the opening; a fluid recovery passage extending into the expansion element through the lumen of the main tube, a distal end of the fluid recovery passage being communicated with a recovery hole at the distal end of the main tube, and the cryogenic fluid flowing into the fluid recovery passage through the recovery hole; wherein the opening is linearly disposed in a length direction of the distal end of the main tube. With the cryoablation catheter according to the present invention, an ablation line may be formed on tissue to be ablated, and through single entirely-lined ablation or a few times of entirely lined continuous ablation, the one-time success rate of ablation is close to the success rate of a surgery.

## Description

### TECHNICAL FIELD

The present invention relates to a cryoablation catheter.

### BACKGROUND

Atrial Fibrillation (AF) is one of the most common clinical tachyarrhythmias. Currently, catheter ablation is the most widely used method for treating AF in China and other countries, intended to cut off myocardial tissue or a bypass which interferes with a normal cardiac electrical activity to restore normal cardiac electrical conduction. The catheter ablation currently in use includes radio-frequency ablation catheter treatment and cryoablation catheter treatment.

A radio-frequency ablation catheter is unable to realize linear ablation during treatment, and forms point-shaped ablation during single ablation or several times of ablation. Such ablation tends to generate gaps, which may cause some tissue not to be ablated, so that the ablation is not thorough, which is not easily observed by a physician. After a period of time, the gap may restore conduction and AF may recur.

In recent years, a cryoablation balloon catheter has become a new technology in the field of AF treatment, and clinical researches prove that cryoballoon ablation for AF is a safe and effective treatment method. Only a balloon-type cryoablation catheter available from Medtronic is mature now. However, a balloon at a distal end of the catheter has a spherical structure or a roughly spherical shape, only suitable for ablation of the pulmonary vein ostium but unable to be used for other tissue regions requiring linear ablation.

Therefore, there is a need for a new cryoablation catheter.

### SUMMARY

According to an aspect of the present invention, there is provided a cryoablation catheter, including: a main tube having a distal end, a proximal end, and at least one lumen; a main-tube fluid delivery tube extending into an expansion element through the lumen of the main tube, an opening being provided at a distal end of the fluid delivery tube, and a cryogenic fluid being ejected out through the opening; a fluid recovery passage extending into the expansion element through the lumen of the main tube, the fluid recovery passage being communicated with a recovery hole at the distal end of the main tube, and the cryogenic fluid flowing into the fluid recovery passage through the recovery hole; wherein the opening is linearly disposed in a length direction of the distal end of the main tube.

According to another aspect of the present invention, there is provided a cryoablation catheter, including: a catheter body having a distal end, a proximal end, and at least one lumen; a headend tube, the distal end of the catheter body being connected with the headend tube; an expansion element, with which the headend tube is sleeved; a fluid delivery tube extending into the expansion element through the lumen of the catheter body, an opening being provided at a distal end of the fluid delivery tube, and a cryogenic fluid being ejected out through the opening; a fluid recovery tube extending into the expansion element through the lumen of the catheter body, a recovery hole being provided at a distal end of a fluid discharge tube, and the cryogenic fluid flowing into the fluid recovery tube through the recovery hole; and a pull wire extending within the lumen of the catheter body with a distal end thereof secured to a distal end of the cryoablation catheter, an axial movement of the pull wire deflecting the distal end of the cryoablation catheter; wherein the opening is linearly disposed in a length direction of the headend tube.

Since the distal end of the fluid delivery tube of the cryoablation catheter according to the present invention has the opening disposed linearly, a substantially linear ablation line may be formed by a single cryogenic operation. For example, the opening at the distal end of the fluid delivery tube is configured as a small hole or slit, and preferably, a plurality of small holes or slits, such as 5, 10, or even more, may be arranged linearly, such as in a straight line or a curved line. The opening may be either linearly provided directly on the fluid delivery tube, for example, when the fluid delivery tube is inserted directly into the headend tube or the expansion element or extends outside the headend tube; or provided linearly when the fluid delivery tube is wound around the headend tube.

In a preferred embodiment of the cryoablation catheter according to the present invention, the distal end of the catheter is moved during ablation to ablate a roof, the isthmus and a posterior wall of the left atrium, thereby forming several electrically isolated regions by division and forming an ablation line at each of these sites. With one-time entirely lined continuous ablation or a few times of entirely lined continuous ablation, the one-time success rate of the ablation is close to the success rate of a surgery.

### BRIEF DESCRIPTION OF DRAWINGS

FIG. 1 shows a schematic structural diagram of a cryoablation catheter according to the present invention;
FIG. 2 is an enlarged view of part I of FIG. 1;
FIG. 3 shows a cutaway view taken along line A-A of FIG. 2;
FIG. 4 is a cutaway view taken along line B-B of FIG. 2;
FIG. 5 shows a schematic diagram of an internal structure of an expansion element according to an embodiment of the present invention;
FIG. 6 shows a schematic diagram of an internal structure of an expansion element according to another embodiment of the present invention;
FIG. 7 shows a schematic structural diagram of a distal end of the ablation catheter;
FIG. 8 shows an enlarged view of part I of FIG. 7;
FIG. 9 shows a schematic structural diagram of the distal end of the ablation catheter;
FIG. 10 shows a schematic deflection diagram of the distal end of the ablation catheter;
FIG. 11 is a schematic diagram of an internal structure of an expansion element according to yet another embodiment of the present invention;
FIG. 12 shows a sectional view of a headend tube according to another embodiment of the present invention;
FIG. 13 shows a schematic structural diagram of a distal end of a cryoablation catheter according to another embodiment of the present invention;
FIG. 14 is a cutaway view taken along line A-A of FIG. 13;
FIG. 15 is a cutaway view of a distal structure of the cryoablation catheter of FIG. 13; and
FIG. 16 is a perspective cutaway view of the distal structure of FIG. 15.

### DESCRIPTION OF EMBODIMENTS

Technical solutions of the present invention will be described in further detail below with reference to exemplary embodiments and accompanying drawings, but the present invention is not limited to the following embodiments.

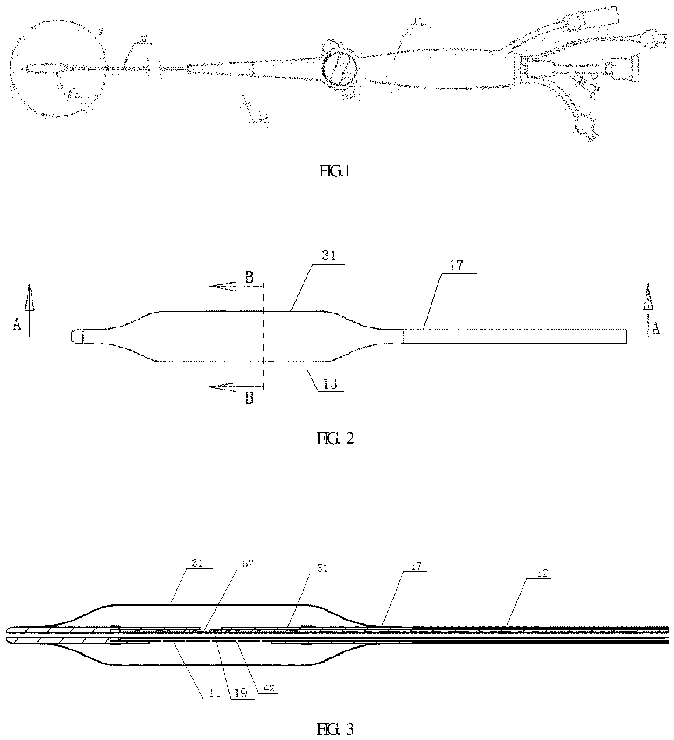

FIG. 1 shows a schematic structural diagram of a cryoablation catheter 10 according to an exemplary embodiment of the present invention; FIG. 2 is an enlarged view of part I in FIG. 1. As shown in FIGS. 1 and 2, the catheter 10 includes a main tube 12 having a distal end, a proximal end and at least one lumen. The main tube may also be referred to as a catheter body or catheter main body. In the present invention, the distal and proximal ends are described relative to an operator; that is, during an operation, the end close to the operator is the proximal end, and the end apart from the operator is the distal end. A treatment element 13 is provided at the distal end of the main tube 12, and a control handle 11 is provided at the proximal end of the main tube 12. The treatment element 13 may be configured as an expansion element 31, such as a balloon, or other suitable elements. According to an embodiment of the present invention, the treatment element 13 includes at least one balloon. Usually, the distal end refers to the end apart from the operator or control handle 11 and the proximal end refers to the other end close to the operator or control handle 11.

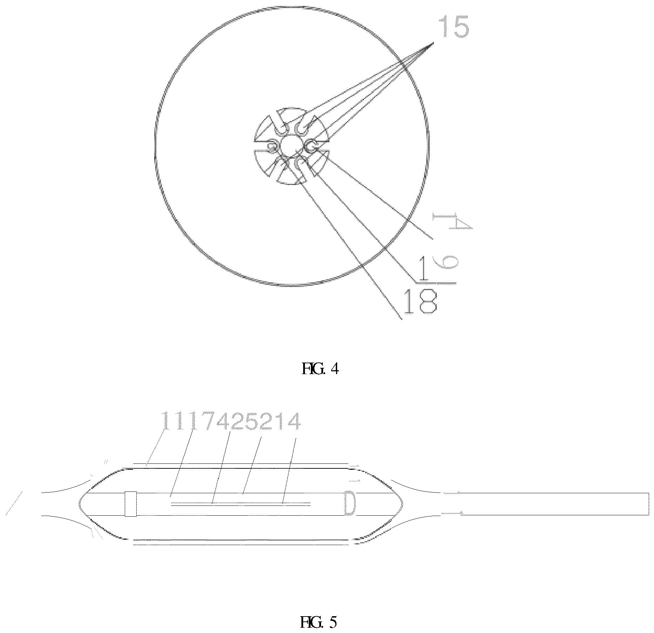

FIG. 3 shows a cutaway view taken along line A-A of FIG. 2; FIG. 4 is a cutaway view taken along line B-B of FIG. 2. As shown in FIGS. 3 and 4, the distal end of the main tube may be provided with a headend tube 17, and the headend tube 17 is sleeved with the expansion element 31, two ends thereof being fixed on the headend tube 17 using a suitable method, such as adhesion. The headend tube 17 is provided at the distal end of the main tube 12. According to an embodiment of the present invention, the headend tube may be omitted, and the expansion element 31 may be directly provided at the distal end of the main tube. According to another embodiment of the present invention, the headend tube 17 is integrally formed with and serves as a part of the main tube.

According to an embodiment of the present invention, the headend tube may be configured such that the part of the headend tube at which the expansion element is mounted is relatively stiff, but a proximal part of the headend tube (i.e., the part connected to the distal end of the main tube) is relatively flexible, so as to pull a pull wire to deflect a distal end of the catheter. The entire headend tube may also be configured to be relatively flexible. The headend tube may also be configured such that only a section of a tube body fixing the pull wire is relatively flexible.

The main tube 12 may include one or more lumens, such as a fluid delivery lumen, a fluid recovery lumen, a through lumen, or the like. The main tube 12 may also include a central lumen provided therein with one or more channels or pipes, such as a fluid delivery tube, a fluid recovery passage, or the like. The headend tube 17 may also include a plurality of lumens, such as a fluid delivery lumen, a fluid recovery lumen, a through lumen, or the like. The through lumens of the main tube 12 and the headend tube 17 may be configured to pass a guide wire or a mapping catheter. According to an embodiment of the present invention, the headend tube 17 includes a fluid delivery lumen, a fluid recovery lumen 15, and a through lumen 19. A fluid delivery tube 14 extends in the fluid delivery lumen, a fluid recovery passage 51 extends in the fluid recovery lumen 15, and a guide wire extends in the through lumen 19, as shown in FIG. 4. According to another embodiment of the present invention, a circulation recovery tube provided in the central lumen may serve as the fluid recovery passage 51.

The expansion element 31 may have a single-layer, double-layer or multilayer structure. When having a single-layer structure, the expansion element may be provided with a coating. The coating is mainly used for reducing a friction coefficient of a surface of an instrument, and may reduce the degree of thrombus coagulation on the surface of the instrument. The coating may be provided at any suitable location, such as a surface of the expansion element. The coating may also be provided at other parts, such as a part of the catheter entering the human body, this part including the expansion element or the main tube, or the like. A main part of the expansion element 31 has a cylindrical structure in an extended state, and two ends have conical structures shrunk to the headend tube 17 and having diameters reduced gradually. According to an embodiment of the present invention, the main part of the expansion element 31 has a diameter of 4-15 mm, and a main straight-line part has a length of 10-30 mm. The expansion element 31 may be in fluid communication with the fluid delivery lumen and the fluid recovery lumen 15 through openings or holes.

FIG. 5 shows a view of an internal structure of the expansion element 31. A distal end of the fluid delivery tube 14 extends through the fluid delivery lumen into the distal end of the main tube or the headend tube 17 located in the expansion element 31, and a tip of the distal end is usually closed, and may be secured in the distal end of the main tube or the headend tube 17 using any suitable methods, such as adhesion. A proximal end of the fluid delivery tube 14 extends out through the control handle to be connected with a cryogenic fluid storage container. An opening 42 is provided at the distal end of the fluid delivery tube 14, and the shape and size of the opening 42 may be set by those skilled in the art as required, for example, the opening 42 may be formed by a plurality of small holes or slits, or by a single slit. Distribution of the plural small holes or slits may also be set by those skilled in the art as required, and the small holes or slits may be linearly arranged, for example, arranged in a straight line or a curve. Centers of the plural small holes or slits may or may not be located on a same straight line. According to an embodiment of the present invention, the centers of the plural small holes or slits are located on the same straight line. The centers of the plural small holes or slits may also be arranged on a substantially or basically straight line as required by those skilled in the art, and a same technical effect may be achieved without departing from the spirit of the present invention. When the opening 42 is configured as a single slit, the single slit may have a straight-line structure or a substantially straight-line structure, or a curved structure.

That is, the opening 42 at the distal end of the fluid delivery tube 14 is configured to be disposed linearly, and formed by arranging the plural small holes or slits, or by the single slit. The linear arrangement means that the opening 42 is in the form of an elongated line or curve, such that a cryogenic fluid may be ejected therefrom to form linear shaped ablation.

A tube body of the distal end of a section of the main tube or the headend tube extending in the expansion element 31 may also be provided with a second opening (not shown) corresponding to the opening 42 in the fluid delivery tube 14. The opening in the headend tube may be formed by a plurality of small holes or slits, or by a large hole or a single slit. That is, the second opening in the distal end of the main tube or the headend tube may be provided by partially cutting the tube body of the distal end of the main tube or the headend tube at a position corresponding to the opening 42 in the fluid delivery tube, such that the cut opening in the tube body of the distal end of the main tube or the headend tube corresponds to the opening 42 in the fluid delivery tube 14 respectively; or by overall cutting the tube body of the distal end of the main tube or the headend tube at the position corresponding to the opening 42 in the fluid delivery tube 14, such that the entire opening 42 is exposed.

According to a preferred embodiment of the present invention, a pressure measuring device, which may be configured as a pressure sensor or other suitable devices, is provided inside the expansion element 31 and configured to measure a pressure in the expansion element 31. Since the pressure in the balloon is indicative of a quantity of refrigerants inside the balloon, surveillance of the pressure inside the balloon may guarantee a refrigeration effect. According to an embodiment of the present invention, the pressure measuring device may be provided on a section of the headend tube 17 (if the headend tube is provided) extending in the expansion element 31, or at other suitable locations. According to another embodiment of the present invention, the pressure measuring device is provided in the control handle, and may be provided at any suitable position in the control handle. In this embodiment, a guide tube may be provided in the lumens of the main tube and the headend tube (if the headend tube is provided), and has a distal end extending to any suitable location in the expansion element, such as the proximal end of the expansion element, with a distal opening thereof facing the interior of the expansion element. A proximal end of the guide tube is secured to the control handle, such that the pressure measuring device on the control handle may measure the pressure inside the expansion element.

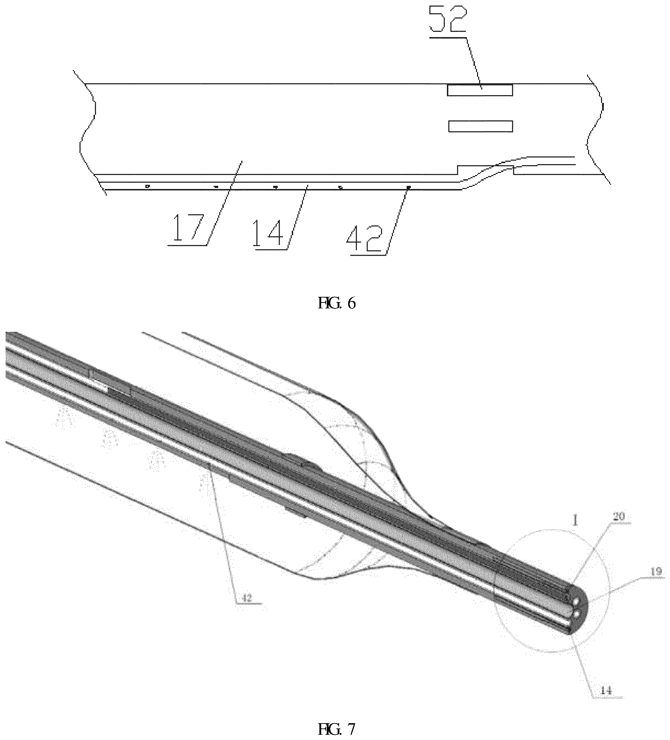

FIG. 6 shows a schematic diagram of an internal structure of the expansion element according to another embodiment of the present invention. As shown in FIG. 6, the distal end of the main tube or the headend tube may also be provided with an opening through which the distal end of the fluid delivery tube extends out; at this point, the distal end of the fluid delivery tube extends in a direction parallel or substantially parallel to the headend tube, and the position of the opening in the headend tube may not correspond to the opening 42 in the fluid delivery tube. At this point, the position of the opening in the headend tube may be set by those skilled in the art according to actual needs without affecting the ejection of the cryogenic fluid through the opening in the fluid delivery tube 14. The fluid delivery tube 14 may or may not be fixed to the headend tube 17. According to an embodiment of the present invention, the fluid delivery tube 14 is fixed to the headend tube 17. A distance between the fluid delivery tube 14 and the headend tube 17 may be set by those skilled in the art according to actual needs.

A distal end of the fluid recovery passage 51 extends into the section of the headend tube 17 located within the expansion element 31, has a normally closed tip, and may be secured to the headend tube 17 using any suitable method, such as adhesion. A proximal end of the fluid recovery passage 51 extends out through the control handle to be connected with the cryogenic fluid storage container and a pressure control unit. The distal end of a section of the fluid recovery passage extending in the expansion element 31 is communicated with a recovery hole 52 at the distal end of the main tube, and the vaporized cryogenic fluid enters the fluid recovery passage 51 through the recovery hole 52. A small hole may be formed in the tube body of the headend tube 17 extending in the expansion element 31 and corresponds to the recovery hole 52; or a part of the tube body of the headend tube corresponding to the recovery hole 52 is directly cut to expose the recovery hole 52, such that gasified cryogenic fluid conveniently enters the fluid recovery passage 51 through the recovery hole 52. A plurality of recovery holes may be provided, so as to discharge the gasified cryogenic fluid.

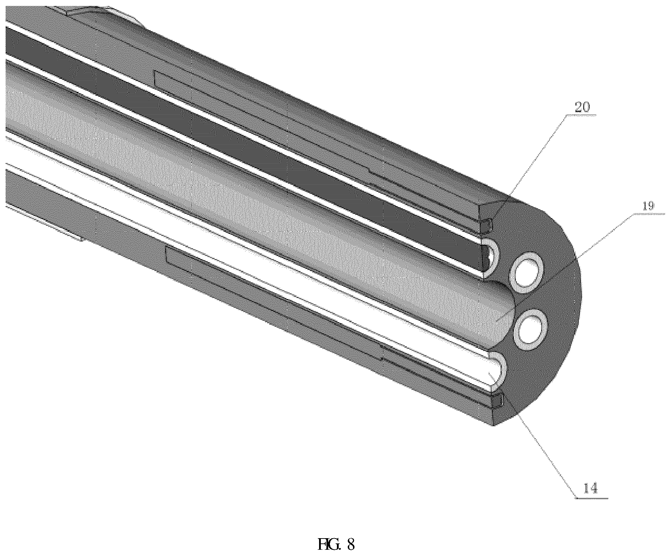

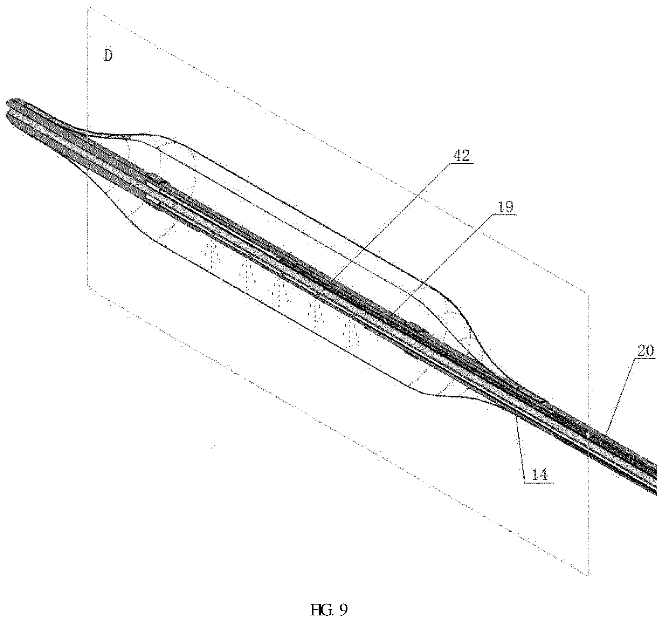

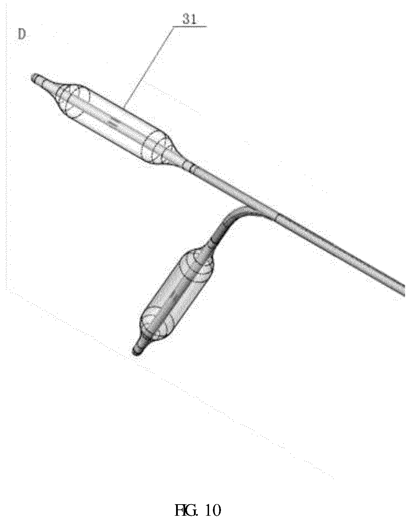

FIG. 7 shows a schematic structural diagram of the distal end of the ablation catheter, and FIG. 8 shows an enlarged view of part I of FIG. 7; FIG. 9 shows a schematic structural diagram of the distal end 13 of the ablation catheter; FIG. 10 shows a schematic deflection diagram of the distal end 13 of the ablation catheter. As shown in FIGS. 7, 8, 9, and 10, the main tube of the cryoballoon catheter 10 includes a through lumen 19 configured to guide and separate a mapping catheter from the fluid delivery tube 14 for the cryogenic fluid. The pull wire 20 may extend within one lumen of the main tube 12. A distal end of the pull wire extends to the distal end of the main tube 12 and is secured by suitable means, such as welding. The pull wire may be fixed at the proximal end of the expansion element near the distal end of the main tube 12.

The fixed position of the pull wire may be selected such that the cryogenic fluid is ejected from the opening 42 in the distal end of the fluid delivery tube 14 in a direction located on a plane formed by a direction of deflection of the distal end of the main tube 12. At this point, the cryogenic fluid may be ejected in a direction of deflection of the distal end of the ablation catheter relative to the distal end of the main tube, for example, as shown by a dotted line in FIGS. 7 and 9. The deflection direction in the present invention refers to a normal direction of a bending section formed after the distal end of the main tube is deflected. At this point, the cryogenic fluid may also be ejected in a direction opposite to the direction of deflection of the distal end of the ablation catheter relative to the distal end of the main tube. The pull wire 20 and an axis of the main tube 12 form a plane D, as shown in FIG. 8, the fluid delivery tube 14 and the pull wire 20 and the axis of the main tube 12 are located in the same plane; that is, the fluid delivery tube 14 is located in the plane D formed by the pull wire 20 and the axis of the main tube. The plane formed by the deflection direction, the same as the plane D, is a plane formed by deflection of the expansion element 31 caused by the axial movement of the pull wire 20 when the pull wire 20 is pulled, as shown in FIG. 10. At this point, by pulling the pull wire 20, such that the expansion element 31 surrounds or is fitted to a site to be ablated, the cryogenic fluid is ejected through the opening 42 of the fluid delivery tube 14 in the deflection plane D, so as to create substantially linear ablation.

One or two pull wires may be provided, and a movement thereof in an axial direction of the catheter deflects the distal end of the main tube 12. According to an embodiment of the present invention, two pull wires are provided, and distal ends of the pull wires are fixed such that the axial movements of the two pull wires deflect the distal end of the main tube 12 in two opposite directions respectively. The two pull wires are arranged such that when either pull wire pulls the distal end of the ablation catheter to deflect, the cryogenic fluid may be ejected in the plane formed by the direction of deflection of the distal end of the ablation catheter relative to the distal end of the main tube. That is, when either of the pull wires pulls the distal end of the ablation catheter to deflect, the cryogenic fluid from the corresponding fluid delivery tube may be ejected in the direction of deflection of the distal end of the ablation catheter relative to the distal end of the main tube. When either of the pull wires pulls the distal end of the ablation catheter to deflect, the cryogenic fluid from the other fluid delivery tube apart from this pull wire may be ejected in a direction opposite to the direction of deflection of the distal end of the ablation catheter relative to the distal end of the main tube.

According to a preferred embodiment of the present invention, the distal end of the pull wire may also extend into and be secured within the distal end of the section of the main tube or the headend tube 17 located within the expansion element 31, and the axial movement of the pull wire may also deflect the expansion element 31. Thus, the expansion element 31 may be adapted to a structure of the tissue to be ablated, resulting in a better ablation effect.

As shown in FIG. 3, the distal end of the main tube or the distal end of the headend tube 17 has an open structure, and when necessary, a distal end of the mapping catheter (not shown) may extend into the through lumen of the main tube 12 through the control handle and extend to the distal opening through the through lumen of the distal end of the main tube or the headend tube 17, so as to extend out.

The mapping catheter may be configured as an annular mapping catheter or a common mapping catheter. The through lumen of the main tube of the cryoballoon catheter 10 is configured to guide and separate the mapping catheter from the fluid delivery tube for the cryogenic fluid. A mapping electrode is provided at the distal end of the mapping catheter. After the distal end of the mapping catheter extends out of the distal opening of the main tube or the headend tube 17, the cryoballoon catheter 10 is pushed forward, such that the mapping electrode at the distal end of the mapping catheter may map the tissue to be ablated, so as to judge whether ablation is successful.

The expansion element 31 may further be provided therein with a temperature sensor 18 which may be fixed at any suitable location within the expandable element, such as at the distal end of the section of the main tube extending within the expansion element. A proximal end of a lead of the temperature sensor 18 extends through the lumen of the main tube to the control handle and extends out of the control handle for connection with a temperature monitoring device (not shown).

The expansion element 31 may further be provided therein with an impedance sensor (not shown) which may be fixed at any suitable location within the expandable element, such as at the distal end of the section of the main tube extending within the expansion element. A proximal end of a lead of the impedance sensor extends through the lumen of the main tube to the control handle and extends out of the control handle for connection with a monitoring device (not shown). The impedance of the impedance sensor may change upon contact with blood, and therefore, the impedance sensor may be configured to monitor whether the expansion element is ruptured.

The control handle is further provided therein with an photocouplingsensor which may be provided at any proper position in the control handle and configured to monitor whether blood is sucked into the control handle, so as to judge whether a rupture exists at any position of the part of the catheter in the human body. Specifically, the optical coupling sensor has a transmitting end and a receiving end, and an output changes when the transmitting end and the receiving end are blocked by an object. The fluid recovery passage is configured as a transparent tube body in the control handle, the tube body is disposed between the transmitting end and the receiving end, and under normal conditions, the optical coupling sensor may normally receive infrared light; during blood passage, signals of the transmitting end and the receiving end are blocked, such that blood passage is determined.

According to a preferred embodiment of the present invention, in use, the catheter 10 enters a target site in the left atrium for treatment by means of a guide instrument, typically a bending controllable sheath, through puncture of the interatrial septum. The placement and positioning of the expansion element within the left atrium rely primarily on the bending control function of the sheath, and have to be implemented with the aid of the bending control function of the catheter 10 itself in particular situations. Therefore, the catheter 10 may or may not have the bending control function. Preferably, the catheter has the bending control function to adapt to a greater range of left atrial structures of different patients.

According to an embodiment of the present invention, the catheter 10 has no bending control function and is not provided with the pull wire, and the proximal end of the control handle or the main tube is provided with an ejection direction indication for indicating the ejection direction of the fluid. Since the catheter may be used in cooperation with a sheath, the sheath may be configured as the bending controllable sheath with a deflectable distal end, and a bending direction of the distal end of the sheath may be kept consistent with the ejection direction indication in use. According to yet another embodiment of the present invention, the ejection direction indication may be provided on the proximal end of the control handle or the main tube even if the pull wire is provided.

Since the linearly arranged small holes are provided in the fluid delivery tube, when the catheter 10 is in use, the distal expansion element 31 performs linear ablation on the roof, the isthmus and the posterior wall of the left atrium by rotating the control handle 11 and manipulating the pull wire, several electrically isolated regions may be formed by division with ablation lines, and one ablation line is formed at each of these sites. With single ablation or a few times of linear continuous ablation, the one-time success rate of the ablation is close to the success rate of a surgery, and the ablation effect may achieve an effect of a surgical maze procedure, thus increasing the success rate of the surgery.

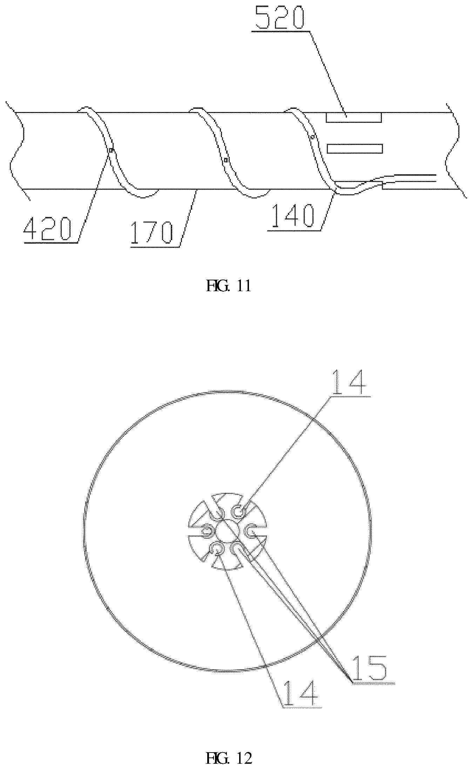

FIG. 11 is a diagram of an internal structure of the expansion element according to another embodiment of the present invention. As shown in FIG. 11, the fluid delivery tube 410 may be wrapped around an outer surface of the headend tube 170, and provided with an opening 420. After the fluid delivery tube 410 is wrapped, the openings in the fluid delivery tube 410 may be arranged in a curve relative to an axis of the fluid delivery tube 410.

That is, the openings 420 in the fluid delivery tube may be arranged such that, as in the previous embodiments, the fluid delivery tube linearly extends in the headend tube, and the openings 420 are linearly arranged on the fluid delivery tube; or the distal end of the fluid delivery tube may extend from the distal end of the headend tube, and at this point, the distal end of the fluid delivery tube extends in a direction parallel to the headend tube; or as in this embodiment, the fluid delivery tube is wrapped around the headend tube, and the openings of the wrapped fluid delivery tube are linearly arranged on the surface of the headend tube.

The remaining structure of the embodiment shown in FIG. 11 is the same as that of FIGS. 1 to 10.

FIG. 12 shows a sectional view of the headend tube 17 according to another embodiment of the present invention. As shown in FIG. 12, the lumens of the headend tube 17 include two fluid delivery lumens and three fluid recovery lumens 15. A fluid delivery tube 14 is provided in each of the two fluid delivery lumens, and has a distal end provided with an opening 42. The cryogenic fluid in the fluid delivery tube 14 may be ejected from the openings 42 of the two fluid delivery tubes 14 simultaneously or separately. By suitably arranging the two pull wires, such that the distal end of the main tube 12 may be deflected in two opposite directions respectively, the ejection direction of the cryogenic fluid is located on a plane formed by the two opposite deflection directions of the distal end of the main tube 12.

The remaining structure of the embodiment shown in FIG. 12 is the same as that of FIGS. 1 to 10.

In the embodiments shown in FIGS. 1 to 10, 11 and 12, the expansion element has a single-layer structure, or a surface of the single-layer structure is coated, and the expansion element may also have a double-layer structure, or a surface of the double-layer structure is coated, which will be described in detail below.

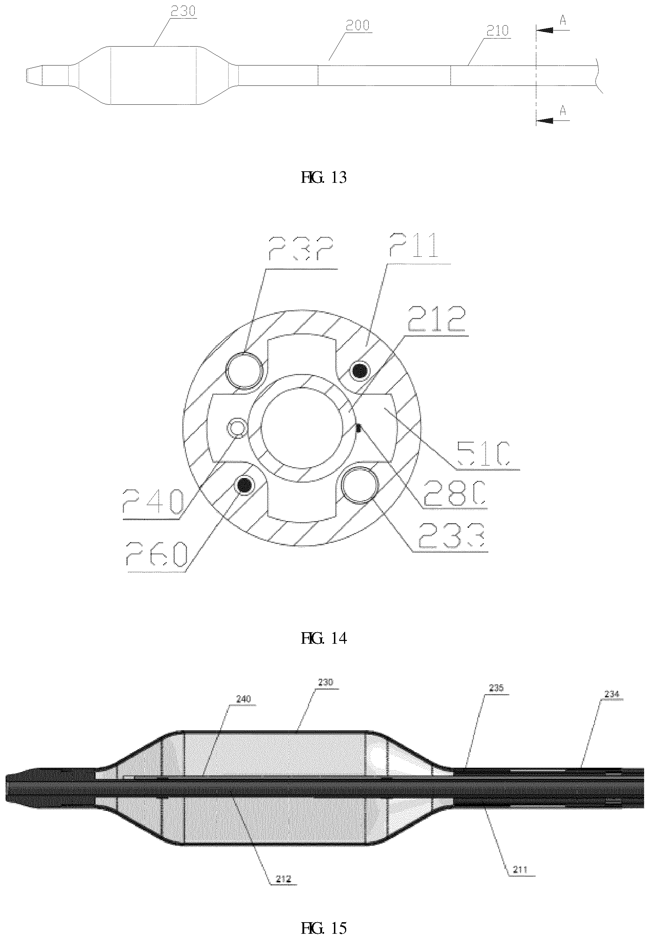

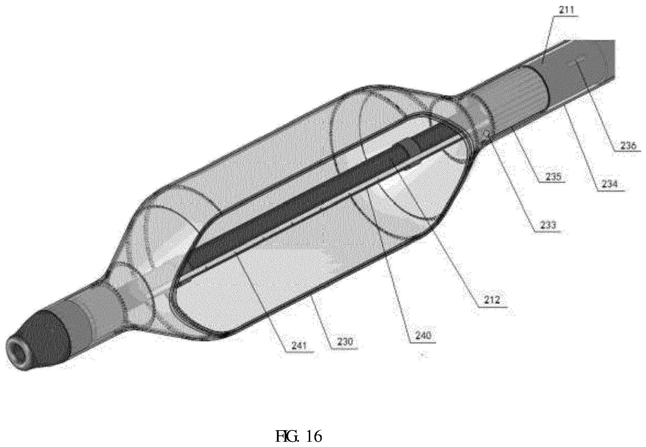

FIG. 13 shows a schematic structural diagram of a distal end of a cryoablation catheter according to another embodiment of the present invention; FIG. 14 is a cutaway view taken along line A-A of FIG. 13; FIG. 15 is a cutaway view of the distal structure of the cryoablation catheter of FIG. 13; and FIG. 16 is a perspective cutaway view of the distal structure of FIG. 15. As shown in FIGS. 13 to 16, the catheter 200 includes a main tube 210 which may be configured as a single-layer tube; the main tube 210 may also include an outer main tube 211 and an inner main tube 212, as shown in FIG. 13. An expansion element 230 is provided at a distal end of the main tube 210. According to an embodiment of the present invention, the expansion element 230 has a distal end provided on the inner main tube 212 and a proximal end provided on a distal end of the outer main tube 121. That is, when two main tubes are provided, the distal end of the outer main tube 211 ends at the proximal end of the expansion element. The outer main tube 211 and the inner main tube 212 may slide relative to each other, and the desired size and shape may be achieved more easily during expansion and contraction of the expansion element. A space between the outer main tube 211 and the inner main tube 212 may serve as a fluid recovery passage 510 for discharging a gasified cryogenic fluid.

The expansion element 230 may have a double-layer structure, and a surface thereof may be coated. The expansion element 230 includes an outer layer 234 and an inner layer 235. Proximal ends of the inner layer 235 and the outer layer 234 of the expansion element may extend to different locations on the outer main tube for fixation, or may extend to the same location for fixation. According to an embodiment of the present invention, the proximal end of the outer layer 234 of the expansion element 230 is closer to the proximal end of the main tube or the headend tube than the proximal end of the inner layer 235, such that a gap exists at the fixation position of the proximal ends of the outer layer and the inner layer of the expansion element 230. By providing a pressure measuring device there, a pressure between the two layers of the expansion element may be monitored. In normal use, the pressure between the two layers of the expansion element is quite low, the pressure inside the inner balloon is relatively high, and when the inner balloon leaks or fails, the pressure may be transmitted between the two layers, therefore whether the inner balloon is broken may be monitored by detecting a change of the pressure from low to high. According to another embodiment of the present invention, a first pressure measuring device may be provided at any suitable position in the control handle, and the pressure between the two layers of the expansion element may be guided to the first pressure measuring device in the control handle via a first guide tube 232 for monitoring the pressure between the two layers. At this point, an opening 236 is provided at a gap at the proximal end of the expansion element 230, i.e., the gap at the fixation position of the proximal ends of the outer layer and the inner layer of the expansion element 230. A distal end of the first guide tube extends through the lumen of the main tube to the opening 236, and a proximal end of the first guide tube extends into the control handle for fixation.

A second pressure measuring device may also be provided at any proper position inside the expansion element and configured to monitor the pressure inside the expansion element. The second pressure measuring device may also be provided in the control handle, at which point the pressure inside the expansion element is guided to the second pressure measuring device at the control handle via a second guide catheter. A distal end of the second guide catheter 233 extends through the lumen of the main tube to the proximal end of the expansion element, and has an opening towards the interior of the expansion element. A proximal end of the second guide catheter 233 extends into the control handle for fixation.

The fluid delivery tube 240 is provided with a small hole 241 which has a same structure as that in the embodiment shown in FIGS. 1 to 10, or the embodiment shown in FIGS. 11 to 12. The pull wire 260, the temperature sensor 280, the impedance sensor, and other structures are the same as those in the embodiment shown in FIGS. 1 to 10, or the embodiment shown in FIGS. 11 to 12.

Embodiments of the present invention are not limited to the above-described examples, and various changes and modifications in form and detail, which may be made by one skilled in the art without departing from the spirit and scope of the present invention, are considered to fall within the scope of the present invention.

## Claims

1. A cryoablation catheter, comprising:
a main tube having a distal end, a proximal end, and at least one lumen;
an expansion element, with which the distal end of the main tube is sleeved;
a fluid delivery tube extending through the lumen of the main tube into the expansion element, an opening being provided at a distal end of the fluid delivery tube, and a cryogenic fluid being ejected out through the opening; and
a fluid recovery passage extending into the expansion element through the lumen of the main tube, a distal end of the fluid recovery passage being communicated with a recovery hole at the distal end of the main tube, and the cryogenic fluid flowing into the fluid recovery passage through the recovery hole; wherein the opening is linearly arranged in a length direction of the distal end of the main tube.

2. The cryoablation catheter according to claim 1, wherein the fluid delivery tube linearly extends within the headend tube, and the opening ia linearly arranged in the fluid delivery tube.

3. The cryoablation catheter according to claim 1, wherein the distal end of the fluid delivery tube extends outside of the headend tube in a direction parallel to the headend tube, and the opening ia linearly provided in the fluid delivery tube.

4. The cryoablation catheter according to claim 1, wherein the distal end of the fluid delivery tube is wrapped around the headend tube, and the opening of the wrapped fluid delivery tube is linearly provided in a surface of the headend tube.

5. The cryoablation catheter according to any one of claims 1 to 4, further comprising a pull wire extending within the lumen of the main tube with a distal end thereof secured to a distal end of the cryoablation catheter, an axial movement of the pull wire deflecting the distal end of the cryoablation catheter.

6. The cryoablation catheter according to claim 5, wherein the pull wire is arranged such that when the pull wire pulls the distal end of the ablation catheter to deflect, the cryogenic fluid may be ejected in a plane formed by a direction of deflection of the distal end of the ablation catheter relative to the distal end of the main tube.

7. The cryoablation catheter according to claim 6, wherein the cryogenic fluid is ejected in the direction of deflection of the distal end of the cryoablation catheter relative to the distal end of the main tube.

8. The cryoablation catheter according to any one of claims 1 to 7, wherein the opening is formed by a plurality of small holes, a plurality of slits, or a single slit.

9. The cryoablation catheter according to claim 8, wherein centers of the plurality of small holes or slits are located at a straight line or substantially a straight line.

10. The cryoablation catheter according to claim 8, wherein the single slit has a shape of a straight line or substantially a straight line.

11. The cryoablation catheter according to claim 9, wherein the plurality of small holes or slits are arranged in one side of a distal tube body of the headend tube, and a straight line of or substantially formed by the centers of the plurality of small holes or slits is parallel or substantially parallel to an axis of the main tube.

12. The cryoablation catheter according to claim 10, wherein the single slit is arranged in one side of the distal tube body of the headend tube, and parallel or substantially parallel to the axis of the main tube.

13. The cryoablation catheter according to claim 8, wherein the centers of the plurality of small holes or slits are substantially located at a curve.

14. The cryoablation catheter according to claim 8, wherein the single slit is curvilinear.

15. The cryoablation catheter according to any one of claims 1 to 7, comprising two fluid delivery tubes and two pull wires, the openings in the two fluid delivery tubes forming two substantially parallel lines respectively.

16. The cryoablation catheter according to claim 15, wherein the two pull wires are arranged such that when either of the pull wires pulls the distal end of the ablation catheter to deflect, the cryogenic fluid may be ejected in the plane formed by the direction of deflection of the distal end of the ablation catheter relative to the distal end of the main tube.

17. The cryoablation catheter according to claim 16, wherein the two pull wires are arranged such that when either of the pull wires pulls the distal end of the ablation catheter to deflect, the cryogenic fluid from the corresponding fluid delivery tube is ejected in the direction of deflection of the distal end of the ablation catheter relative to the distal end of the main tube.

18. The cryoablation catheter according to any one of the preceding claims, wherein the expansion element has a single-layer structure; preferably, the single-layer expansion element is provided with a coating.

19. The cryoablation catheter according to any one of the preceding claims, wherein the expansion element has a double-layer structure comprising an inner layer and an outer layer; preferably, the double-layer expansion element is provided with a coating.

20. The cryoablation catheter according to claim 19, wherein the main tube comprises an outer main tube and an inner main tube.

21. The cryoablation catheter according to claim 20, wherein proximal ends of the outer layer and the inner layer of the expansion element extend to different locations on the outer main tube for fixation, and a gap exists at a fixation position of the proximal ends of the outer layer and the inner layer of the expansion element.

22. The cryoablation catheter according to any one of the preceding claims, wherein the expansion element has a diameter of 4-15 mm.

23. The cryoablation catheter according to any one of the preceding claims, wherein a main straight-line part of the expansion element has a length of 10-30 mm.

24. The cryoablation catheter according to any one of the preceding claims, wherein a pressure measuring device is provided within a balloon.

25. The cryoablation catheter according to claim 21, wherein a pressure measuring device is provided in the gap at the fixation position of the proximal ends of the outer layer and the inner layer of the expansion element.

26. The cryoablation catheter according to any one of the preceding claims, wherein a pressure measuring device is provided within a control handle.

27. The cryoablation catheter according to claim 26, wherein a guide tube is provided within the lumen of the main tube, and has a distal end extending into the expansion element.

28. The cryoablation catheter according to claim 26, wherein an opening is provided in the gap at the fixation position of the proximal ends of the outer layer and the inner layer of the expansion element, a distal end of a first guide tube extends through the lumen of the main tube to the opening, and a proximal end of the first guide tube extends into the control handle for fixation.

29. The cryoablation catheter according to claim 28, wherein a distal end of a second guide catheter extends through the lumen of the main tube to the proximal end of the expansion element, and has an opening towards the interior of the expansion element, and a proximal end of the second guide catheter extends into the control handle for fixation.

30. The cryoablation catheter according to any one of the preceding claims, wherein the control handle or the main tube is provided with an ejection direction indication.

31. The cryoablation catheter according to any one of the preceding claims, wherein the pull wire extends within a tube body of the main tube and has a distal end secured to the distal end of the main tube.

32. The cryoablation catheter according to any one of the preceding claims, wherein the distal end of the pull wire extends into a section of the main tube or the inner main tube located within the expansion element for fixation.

33. The cryoablation catheter according to any one of the preceding claims, wherein a second opening is provided in the section of the main tube or the inner main tube extending within the expansion element and corresponds to the opening in the fluid delivery tube.

34. The cryoablation catheter according to claim 33, wherein the second opening in the main tube or the inner main tube is formed by a plurality of small holes, a plurality of slits, a large hole or a single slit.

35. The cryoablation catheter according to any one of the preceding claims, wherein an impedance sensor is provided within the expansion element.

36. The cryoablation catheter according to any one of the preceding claims, wherein an optical coupling sensor is provided within the control handle.

37. A cryoablation catheter, comprising: a catheter body having a distal end, a proximal end, and at least one lumen; a headend tube, the distal end of the catheter body being connected with the headend tube; an expansion element, with which the headend tube is sleeved; a fluid delivery tube extending into the expansion element through the lumen of the catheter body, an opening being provided at a distal end of the fluid delivery tube, and a cryogenic fluid being ejected out through the opening; and a fluid recovery tube extending into the expansion element through the lumen of the catheter body, a recovery hole being provided at a distal end of a fluid discharge tube, and the cryogenic fluid flowing into the fluid recovery tube through the recovery hole; wherein the opening is linearly disposed in a length direction of the headend tube.

38. The cryoablation catheter according to claim 37, further comprising a pull wire extending within the lumen of the catheter body with a distal end thereof secured to a distal end of the cryoablation catheter, an axial movement of the pull wire deflecting the distal end of the cryoablation catheter.

39. The cryoablation catheter according to claim 37, wherein the fluid delivery tube linearly extends within the headend tube, and the opening is linearly provided in the fluid delivery tube.

40. The cryoablation catheter according to claim 38, wherein the pull wire is arranged such that when the pull wire pulls the distal end of the ablation catheter to deflect, the cryogenic fluid may be ejected in a plane formed by a direction of deflection of the distal end of the ablation catheter relative to the distal end of the main tube.

41. The cryoablation catheter according to claim 40, wherein the cryogenic fluid is ejected in the direction of deflection of the distal end of the cryoablation catheter relative to the distal end of the main tube.
